# EUROPÄISCHE PATENTANMELDUNG

(11) **EP 4 056 222 A1**
(43) Veröffentlichungstag der Anmeldung: **14.09.2022**
(21) Anmeldenummer: 21161564.6
(22) Anmeldetag: 09.03.2021
(51) Int. Cl.: A61M 25/09, A61M 25/00

(54) **DRAHTVORRICHTUNG UND VERFAHREN ZUM SELEKTIVEN VERSTEIFEN EINER DRAHTVORRICHTUNG**

(71) Anmelder: SoftRail Medical AG, 8500 Frauenfeld (CH)
(72) Erfinder: Gerig, Thomas, 3400 Burgdorf (CH); von Weymarn, Alexander, 8500 Frauenfeld (CH)
(74) Vertreter: Hepp Wenger Ryffel AG

(57) **Zusammenfassung**

Die Erfindung betrifft eine Drahtvorrichtung, insbesondere Drahtvorrichtung zum Einführen in einen Körpergang, mit einem Versteifungsabschnitt mit einem Druckschlauch (2), einer Versteifungsschicht (3) und einem Aussenschlauch (4). Die Versteifungsschicht ist konzentrisch um den Druckschlauch angeordnet, und der Aussenschlauch konzentrisch ausserhalb der Versteifungsschicht angeordnet. Der Aussenschlauch ist radial steif ausgebildet und die Versteifungsschicht radial bewegbar, ausgebildet. Im Druckschlauch ist derartig ein Druck aufbaubar, dass die Versteifungsschicht von innen gegen den Aussenschlauch drückbar ist und so unter Druck der Versteifungsabschnitt reversibel versteifbar ist. Der Druck im Druckschlauch ist insbesondere abbaubar, so dass der Versteifungsabschnitt wieder flexibel ist.

## Beschreibung

Die Erfindung richtet sich auf eine Drahtvorrichtung und ein Verfahren zum selektiven Versteifen einer Drahtvorrichtung.

Bei minimal-invasiven operativen Verfahren ist die Verwendung einer Drahtvorrichtung üblich. Eine solche Drahtvorrichtung kann beispielsweise zum Führen eines Katheters verwendet werden, insbesondere in engen Gefässen. Zu diesem Zweck sollte eine Drahtvorrichtung Drehmomentübertragung erlauben.

Eine solche Drahtvorrichtung kann unter anderem zur Entfernung eines Thrombus Verwendung finden. Zu diesem Zweck kann der Thrombus durchstossen und anschliessend durch einen Katheter aspiriert werden.

In vielen Fällen müssen solche Drahtvorrichtungen, oftmals lange Strecken im Körper des Patienten zurücklegen, Windungen und Winkel entlang der Blutgefässe vollführen können und selbst in kleine Blutgefässe einführbar sein.

Ausserdem sollte eine Verwendung der Drahtvorrichtung in Kombination mit Werkzeugen und/oder operativen Behandlungselementen wie Stents, Coils oder ähnlichem möglich sein.

Bei diesen medizinischen Behandlungen ist es besonders wichtig möglichst präzise und minimal-invasiv arbeiten zu können, da das Leben des Patienten in Gefahr sein kann.

Da die Drahtvorrichtung elastisch verformbar sein muss, um sich den Gefässwindungen anzupassen, gesteuert und eingeführt werden zu können, kann die Drahtvorrichtung sehr leicht abrutschen oder abgelenkt werden. Dies ist zum einen sehr gefährlich, da somit nur schwer präzise gearbeitet werden kann und die Behandlung in die Länge gezogen werden kann. Zum anderen besteht die Gefahr, dass der Patient Schaden erleidet oder sogar stirbt. Beispielsweise könnte ein Aneurysma aufreissen, oder es könnten in den Gefässen vorhandene Kalkablagerungen, durch ungünstiges Verrutschen lösen und schwere Schäden verursachen.
Vielfach muss während der Behandlung auf eine andere Drahtvorrichtung gewechselt werden, so dass der Materialverbrauch sehr hoch ist und somit auch die Kosten.

Es ist daher Gegenstand der vorliegenden Erfindung, diese Nachteile des Standes der Technik zu überwinden und eine Drahtvorrichtung vorzustellen, mit der minimal-invasive operative Eingriffe mit möglichst geringem Risiko, schnell ausgeführt werden können und die Kosten gering gehalten werden können.

Die Aufgabe wird durch eine Drahtvorrichtung und ein Verfahren gemäss den unabhängigen Ansprüchen gelöst.

Insbesondere wird die Aufgabe durch eine Drahtvorrichtung, insbesondere Drahtvorrichtung zum Einführen in einen Körpergang, gelöst, die einen Versteifungsabschnitt mit einem Druckschlauch, einer Versteifungsschicht und einem Aussenschlauch umfasst. Die Versteifungsschicht ist konzentrisch um den Druckschlauch angeordnet, und der Aussenschlauch ist konzentrisch ausserhalb der Versteifungsschicht angeordnet. Der Aussenschlauch ist radial steif ausgebildet und die Versteifungsschicht radial bewegbar ausgebildet. Im Druckschlauch ist derartig ein Druck aufbaubar, dass die Versteifungsschicht von innen gegen den Aussenschlauch drückbar ist und so unter Druck der Versteifungsabschnitt reversibel versteifbar ist. Insbesondere ist der Druck im Druckschlauch abbaubar, so dass der Versteifungsabschnitt wieder flexibel ist.

Die Drahtvorrichtung hat somit den Vorteil, dass sie selektiv reversibel versteift werden kann.

Der bevorzugte Druck kann im Wesentlichen in einem Bereich von 3-40 bar, insbesondere im Wesentlichen bei 16 bar, liegen. Auch andere Druckbereiche sind vorstellbar, solange eine ausreichende Versteifung gewährleistet ist.

Radial steif im Sinne der Erfindung heisst, dass die radial steife Schicht nur minimal durch Druck in radialer Richtung erweiterbar ist und so überhaupt ein Druck aufgebracht werden kann. Radial bewegbar heisst, dass die Schicht nicht radial steif ausgebildet ist. Radial steif schliesst nicht aus, dass die Schicht in anderen Richtungen flexibel ausgebildet ist.

Der Druck kann vorzugsweise durch eine externe Vorrichtung, die weitere Schutzmassnahmen beinhalten kann, wie beispielsweise das Abschalten bei Druckabfall durch ein Leck, aufgebracht, überprüft und abgelassen werden. Alternativ kann der Druck auch manuell angelegt werden.

An dieser Stelle ist anzumerken, dass die Druckdifferenz zum menschlichen Körper berücksichtigt werden muss. Deshalb ist die Drahtvorrichtung so ausgebildet, dass ein hoher Druck zu- und abgeführt werden kann. Bevorzugt wird ein Druck oberhalb des atmosphärischen Druckes verwendet und kein Vakuum bzw. Unterdruck. Dies hat den Vorteil, dass die Druckdifferenz viel höher ausfällt, da der Unterdruck auf einen Maximalwert von 1 bar beschränkt ist.

Die Ausbildung einer Versteifung des Versteifungsabschnitts erleichtert das Fixieren der Drahtvorrichtung an der gewünschten Stelle und erlaubt einen präziseren Eingriff, der die Sicherheit des Patienten erhöht, praktikabler durchführbar ist und ein schnelleres Arbeiten gewährleistet.

Eine Drahtvorrichtung ist vorzugsweise so ausgebildet, dass eine Vorrichtung, insbesondere ein Katheter, auf sie aufgeschoben werden kann. Der Draht ist vorzugsweise mittig in einem solchen Katheter angeordnet und wird von dem Katheter ganz oder zumindest teilweise umschlossen.
Somit eignet sich die Drahtvorrichtung vorzugsweise um einen Katheter in Gefässen zu führen, indem die Drahtvorrichtung Biegungen in den Gefässen vollziehen kann.
Im versteiften Zustand, kann der Druck zudem wieder reduziert werden und die Versteifung rückgängig gemacht werden.

Es können einzelne Bereiche, wie ein vorderer Teil, ein hinterer Teil und/oder ein mittlerer Teil der Drahtvorrichtung versteifbar ausgebildet sein. Der Versteifungsabschnitt der Drahtvorrichtung kann also die vollständige Drahtvorrichtung umfassen oder nur einen Teilabschnitt. Sofern nur ein Teilabschnitt der Drahtvorrichtung durch den Versteifungsabschnitt gebildet wird, ist der Rest der Drahtvorrichtung vorzugsweise nicht versteifbar ausgebildet.

Im Innersten der Drahtvorrichtung ist ein Innenraum, der von dem Druckschlauch umgeben ist. Der Innenraum wird im ordnungsgemässen Einsatz vorzugsweise nicht durch Knicke oder Druck auf die Drahtvorrichtung verkleinert.
Der Innenraum weist vorzugsweise zum distalen Ende der Drahtvorrichtung eine Öffnung auf und ist vorzugsweise am proximalen Ende der Drahtvorrichtung, welches in die Gefässe eingeführt wird, verschlossen. Der Innenraum des Druckschlauchs kann auch vor dem proximalen Ende verschlossen sein.

In diesem Zusammenhang ist die Ausbildung von mehreren Versteifungabschnitten in einer Drahtvorrichtung vorstellbar. Der Druckschlauch erstreckt sich vorzugsweise zumindest vom distalen Ende der Schlauchvorrichtung bis zum proximalen Ende des letzten Versteifungsabschnitts.

Für die Funktion der Drahtvorrichtung ist es wichtig, dass der Aussenschlauch radial steif ausgebildet ist. Diese radiale Steifheit bedingt, dass es zu keinen oder nur kleinen Ausdehnungen des Aussenschlauchs kommt. Somit ist sichergestellt, dass der Aussenschlauch einen Druckaufbau ermöglicht und der Aussenschlauch im Gegensatz zur Versteifungsschicht im Wesentlichen nicht radial beweglich ist.

Dennoch ist eine hohe Flexibilität und Verbiegbarkeit der Drahtvorrichtung im unversteiften Zustand möglich, damit die Drahtvorrichtung Windungen entlang der Gefässe und Verzweigungen der Gefässe vollziehen kann.

Die radiale Beweglichkeit der Versteifungsschicht ist hingegen wichtig, damit der Versteifungsabschnitt durch Druck versteift werden kann. Die Versteifung ist durch Formschluss und/oder Kraftschluss möglich, wenn die Versteifungsschicht durch den aufgebauten Druck von innen gegen den Aussenschlauch gedrückt werden kann.

Der Druckschlauch der Drahtvorrichtung kann erweiterbar, insbesondere elastisch und/oder auffaltbar, ausgebildet sein.

Der Druckschlauch kann Druck ausgesetzt werden und somit die Versteifungsschicht gegen den Aussenschlauch drücken und den Versteifungsabschnitt der Drahtvorrichtung versteifen. Diese Anordnung bietet den Vorteil, dass eine einfache selektive Versteifbarkeit in miniaturisierter Bauweise möglich wird.

Es ist anzumerken, dass diese Anordnung vorteilhaft für potentiell auftretende Lecks im Druckschlauch ist. Die Sicherheit des Patienten wäre trotz eines Lecks gewährleistet, da der Druck im Druckschlauch aufgebaut wird. Es müsste also ein Leck im Druckschlauch und im Aussenschlauch auftreten, damit der Druck über den Aussenschlauch entweichen könnte.
Vorzugsweise ist der Aussenschlauch druckdicht ausgebildet.

Ausserdem kann somit ein Druck auf die Versteifungsschicht ausgeübt werden, ohne dass die Versteifungsschicht eine druckdichte Schicht aufweisen müsste. Dies kann den Widerstand gegenüber radialer Beweglichkeit verringern.

Es ist auch eine Versteifungsschicht aus spiralförmig angeordneten Elementen vorstellbar. Zudem ist eine Versteifungsschicht vorstellbar, die einen Verschluss mit einer komplementär ausgebildeten Innenoberfläche des Aussenschlauchs zum Versteifen der Drahtvorrichtung bildet.

Die Versteifungsschicht ist vorzugsweise dennoch nicht monolithisch gefertigt.

Diese bevorzugte Gestaltung der Drahtvorrichtung ermöglicht eine flexiblere Ausgestaltung der Versteifungsschicht, da sie eine nicht geschlossene, perforierte, gerillte, spiralförmige, maschenförmige, verflochtene und/oder netzförmige Struktur, Konstruktion und/oder Oberfläche, aufweisen kann.

Dies kann sich vorteilhaft auf die Versteifung des Versteifungsabschnitts auswirken, da der Versteifungsabschnitt somit auch leichter während starker Verbiegung versteift werden kann.
Der Druckschlauch kann expandieren, wenn er Druck ausgesetzt wird, und umfasst vorzugsweise elastisch verformbares Material, und/oder einen faltbares Material, beispielsweise einen Kunststoff.
Besonders geeignet für einen auf- und einfaltbaren Druckschlauch sind Nylon, Polyethylenterephthalat und/oder Polyether-Block-Amid.

Insbesondere vorteilhafterweise umfasst der Druckschlauch dehnbare Elastomere oder anderes thermoplastisches Kunststoffmaterial, wie beispielsweise Silikon und/oder thermoplastische Elastomere und/oder faltbare Kunststoffe. Diese Stoffe weisen eine hohe Biokompatibilität auf, wirken nicht toxisch, sind kostengünstig und sind vorzugsweise elastisch sehr gut verformbar.

Besonders vorteilhaft ist ein Druckschlauch, der einen zusammengefalteten Zustand ohne Einwirkung von zusätzlichem Druck aufweisen kann und einen entfalteten Zustand beim Aufbringen eines Druckes aufweisen kann. Durch einen solchen auffaltbaren Druckschlauch ist eine fest vorgegebene Maximal- und Minimalgrösse beim Anlegen ordnungsgemässer Drücke gewährleistet und es kann eine Versteifung mit maximierter Dichtheit gewährleistet werden.

Die Versteifungsschicht kann zumindest zwei Stabilisierungselemente umfassen. Die Ausrichtung der Stabilisierungselemente kann zumindest einen Teilvektor in Längsrichtung der Drahtvorrichtung aufweisen. Die Stabilisierungselemente sind vorzugsweise gegeneinander verschiebbar.

Die einzelnen Stabilisierungselemente können im nicht versteiften Zustand gegeneinander beweglich, insbesondere verschiebbar, sein. Bevorzugt sind die Stabilisierungselemente wenig zugelastisch und vorzugsweise zudem wenig druckelastisch in Längsrichtung.

Die Stabilisierungselemente der Versteifungsschicht sind vorzugsweise in höherem Mass entlang der Längsrichtung ausgebildet, sodass der Winkel der Stabilisierungselemente zwischen Längsachse der Drahtvorrichtung und Stabilisierungselement in einem Bereich zwischen -45° und 45°, insbesondere zwischen -30° und 30°, ausgebildet ist. Es ist auch möglich verschiedene Ausrichtungen, also zwei oder mehr unterschiedliche Winkel zur Längsachse der Stabilisierungselemente bezüglich der Längsachse vorzusehen. Dies hat den Vorteil, dass die Verbiegbarkeit nur gering eingeschränkt wird, aber der Formschluss während der Versteifung in Längsrichtung zwischen der Versteifungsschicht und dem Aussenschlauch und vorzugsweise dem Druckschlauch verstärkt wird.

Die Stabilisierungselemente sind vorzugsweise so geformt, dass sie durch hohen Druck in Richtung des Aussenschlauchs eine hohe Reibung mit dem Aussenschlauch aufweisen und zu einer Versteifung der Drahtvorrichtung führen können.

Die Versteifungsschicht und/oder Stabilisierungselemente umfassen vorzugsweise Metalle und/oder Kunststoffe.
Insbesondere geeignet sind beispielsweise Edelstahl oder Polyamid.

Dieser Zusammenhalt zwischen den einzelnen Stabilisierungselementen der Versteifungsschicht kann durch mechanische Adhäsion, wie formschlüssige Verklammerung in Vertiefungen, und/oder durch spezifische Adhäsion ausgebildet werden.

In diesem Zusammenhang wäre auch die Verwendung von Stabilisierungselementen des Aussenschlauchs oder Verwendung von zusätzlichen Stabilisierungselementen auf der Innenseite des Aussenschlauchs vorstellbar, die zum Versteifen, zum Beispiel durch Formschluss und/oder Kraftschluss, beitragen können.

Die Stabilisierungselemente der Drahtvorrichtung bestehen vorzugsweise aus Metall und/oder Kunststoff.
Insbesondere ist vorzugsweise eine Vielzahl von Stabilisierungselementen zu einem Geflecht verflochten.

Metalle und Kunststoffe haben sehr gute elastische Eigenschaften und sind meistens kostengünstig. Ausserdem ist eine gute Elastizität des Materials wichtig, sodass beim Wegfallen der einwirkenden Kraft durch den Druck die Stabilisierungselemente in die Ursprungsform zurückkehren können.
Somit wird gewährleistet, dass die Drahtvorrichtung versteift werden kann, aber auch wieder elastisch verformbar werden kann, wenn der Druck reduziert wird.
Vorteilhaft ist ebenfalls, wenn keine starke elastische Hysterese des Materials vorliegt, also nach dem Entfernen der auslenkenden Kräfte keine Deformation zurückbleiben kann. Einer elastischen Hysterese wird durch die Struktur eines Geflechts, Verwendung eines elastischen Materials und/oder einer nicht plastischen Verformung entgegengewirkt.

Die Elastizität der Versteifungsschicht kann anstatt oder zusätzlich zur Materialelastizität auch durch die Art der Versteifungsschicht erzielt werden. Ein relativ lockeres Geflecht kann durch Aufbringen eines Druckes eine gewisse elastische radiale Verformung erzielen auch wenn das Material des Geflechtes an sich nicht oder kaum elastisch ist.

Die Verwendung von Stabilisierungselementen aus Metall und/oder Kunststoff erlaubt zudem eine kostengünstige Herstellung. Ein Geflecht kann maschinell sehr schnell gefertigt werden und verringert somit die Kosten der Erfindung.

Eine besonders vorteilhafte Gestaltung ergibt sich für die oben beschriebenen Winkel zwischen -45° und 45°, insbesondere zwischen -30° und 30°, in Kombination mit einem Geflecht.

Dennoch wären auch andere Herstellungsmöglichkeiten zum Erzeugen einer vorteilhaften Struktur der Stabilisierungselemente, wie Laserschneiden vorstellbar.

Der Aussenschlauch der Drahtvorrichtung ist vorzugsweise druckdicht ausgebildet und insbesondere aus einem Kunststoff und/oder Metall hergestellt.

Der Aussenschlauch weist vorzugsweise eine Wanddicke in einem Bereich von 0.01 bis 0.8 mm , insbesondere von 0.07 mm , auf und kann Materialien wie Edelstahl, Platin, Iridium, Barium, Wolfram und/oder Plastik, insbesondere Thermoplasten wie Silikone, Polyetheretherketon, Fluorpolymere, Polyvinylchlorid und/oder Polyurethan umfassen.

Bei der Wahl des Materials muss darauf geachtet werden, dass sowohl die mechanischen Eigenschaften, in Bezug auf die Flexibilität, die Oberflächenstruktur, in Bezug auf die Thrombogenität, und die Chemie der Oberfläche, in Bezug auf Beschichtbarkeit und Ladung, vorteilhaft ist. Diese vorteilhaften Eigenschaften weisen die obig genannten Materialien auf und eignen sich somit für die Verwendung in der Drahtvorrichtung.

Vorzugsweise ist der Aussenschlauch zudem armiert, sodass der Querschnitt unter Druck nicht so stark vergrössert und/oder komprimiert wird. Die Drahtvorrichtung weist vorzugsweise eine hohe Druckbeständigkeit auf. Für die Armierung kann der Aussenschlauch durch Spiralen, Maschen, einen Draht oder ein Geflecht mit Querorientierung stabilisiert werden. Die Armierungsstruktur ist vorzugsweise auf ein Verhältnis des Widerstandes der radialen Ausdehnung und longitudinalen Ausdehnung optimiert, indem zum Beispiel die Dicke der Armierung und der Winkel zur Drahtvorrichtung angepasst wird.

Die Wahl dieser Stoffe und einer vorteilhaften Armierung für den Aussenschlauch gewährleistet, dass die Drahtvorrichtung elastisch verformbar ist, aber bei sachgemässer Benutzung nicht zusammengedrückt werden kann. In Längsrichtung sollte der Aussenschlauch zudem vorzugsweise nicht oder kaum elastisch verformbar sein.

Die Drahtvorrichtung kann eine torsionssteife Struktur, bevorzugt einen Hypotube, eine helikale und/oder verdrillte Struktur, umfassen.

Vorzugsweise kann eine Drehung der Drahtvorrichtung im Bereich des distalen Endes der Drahtvorrichtung eine analoge Drehung im Bereich des proximalen Endes der Drahtvorrichtung bewirken.

Somit kann die Drahtvorrichtung in den Gefässen vom distalen Ende, welches vorzugsweise ausserhalb der Gefässe angeordnet ist, gedreht werden.

Die Übertragung des Drehmoments ist vorzugsweise im Wesentlichen 1:1. Die Drahtvorrichtung ist also vorzugsweise so ausgebildet, dass eine Drehung in mindestens eine Richtung der Drahtvorrichtung am distalen Ende um einen Winkel im Wesentlichen dieselbe Drehung um den Winkel am proximalen Ende zur Folge hat.

Eine aufschiebbare Vorrichtung, insbesondere vorzugsweise ein Katheter und/oder Dilatator, kann somit durch die durch die Drahtvorrichtung vorgegebene Struktur steuerbar sein.

Die Drahtvorrichtung kann vorzugsweise durch Versteifung des Versteifungsabschnitts eine bestimmte Ausrichtung ausbilden. Die aufschiebbare Vorrichtung kann entlang dieser Ausrichtung aufgeschoben werden.

Die torsionssteife Struktur der Drahtvorrichtung ist vorzugsweise so ausgebildet, dass das Drehmoment um die Längsachse der Drahtvorrichtung entlang der Drahtvorrichtung in zumindest eine Richtung übertragbar ist. Eine richtungsabhängige Übertragung von Drehmoment der Drahtvorrichtung kann durch eine bestimmte Wicklung, Armierung, Schnitte und/oder Entfernung von Material ausgebildet sein.

In diesem Zusammenhang ist es vorstellbar, dass die Drehung der Drahtvorrichtung zur Ausrichtung in den Gefässen nur in eine Richtung vornehmbar ist.
Somit kann die in eine Richtung torsionssteife Struktur vorzugsweise mit geringerem Materialverbrauch und/oder dünnschichtiger ausgebildet sein.

Die Drahtvorrichtung kann zudem in beide Drehrichtungen um die Längsachse der Drahtvorrichtung torsionssteif ausgebildet sein.

Die torsionssteife Struktur kann in der Versteifungsschicht und/oder dem Aussenschlauch und/oder dem Druckschlauch angeordnet sein. Es wäre auch die Verwendung der torsionssteifen Struktur als zusätzliche Schicht der Drahtvorrichtung vorstellbar.

Die torsionssteife Struktur kann zudem gute Formerholungseigenschaften aufweisen.
Vorzugsweise ist die Drahtvorrichtung steif gegenüber Knicken und/oder Kompressionen ausgebildet, insbesondere bei Drehung um die Längsachse.

Die torsionssteife Struktur kann Metalle und/oder Kunststoffe umfassen. Insbesondere kann die torsionssteife Struktur Edelstahl, Nickel Titanium, Wolfram, eine Fluorpolymer-Schicht, Allzweckharze, Polyamide und Polyetheretherketone umfassen.

Die torsionssteife Struktur kann durch eine röhrenförmige Struktur ausgebildet sein. Diese röhrenförmige Struktur kann zudem Schnitte und/oder entfernte Flächensegmente aufweisen, insbesondere in regelmässigen und/oder alternierenden Abständen.

Die torsionssteife Struktur kann durch einen Hypotube ausgebildet werden. Der Hypotube kann unter Verwendung von Prozesstechnologien wie Laserschneiden oder Kerndrahtschweissen hergestellt sein.

Die röhrenförmige Struktur kann durch Laserschneiden, insbesondere in Umfangsrichtung, modifiziert sein.

Die torsionssteife Struktur kann durch eine verdrillte und/oder helikale Struktur ausgebildet sein. In diesem Zusammenhang sind helikale Strukturen vorstellbar, die in einer oder beiden Drehrichtungen um die Längsachse der Drahtvorrichtung torsionssteif ausgebildet sind.

Insbesondere eine helikale und/oder zumindest teilweise helikale Anordnung der Schnitte und/oder der entfernten Flächensegmente der torsionssteifen Struktur ist vorstellbar.

Die torsionssteife Struktur kann mehrere Schichten mit anderen Orientierungen aufweisen, welche eine anisotrope Krafteinwirkung der Drahtvorrichtung bewirken.

Die Drahtvorrichtung kann eine Fluidkupplung umfassen, durch die ein Fluid in den Druckschlauch einbringbar ist. Insbesondere weist ein Querschnitt durch die Fluidkupplung maximal dieselbe Ausdehnung wie ein Querschnitt des Versteifungsabschnitts auf.

Die Fluidkupplung ist vorzugsweise dazu ausgebildet einen Druck durch Verbindung mit einer Druckvorrichtung aufbauen und/oder abbauen zu können.
Die Fluidkupplung ist vorzugsweise so ausgebildet, dass ein Fluid durch eine Druckvorrichtung in den Druckschlauch einbringbar ist. Vorzugsweise kann durch die Druckvorrichtung das Fluid wieder aus der Fluidkupplung entfernbar sein.

Die Fluidkupplung weist vorzugsweise eine geringere, oder nur geringfügig grössere, Ausdehnung im Querschnitt auf als der Versteifungsabschnitt.
Dies ermöglicht vorzugsweise, dass eine aufschiebbare Vorrichtung, insbesondere vorzugsweise ein Katheter und/oder Dilatator, über die Drahtvorrichtung schiebbar ist.

Die Fluidkupplung kann Dichtungsvorrichtungen, insbesondere Kunststoffdichtungen aufweisen.

Als konkrete Ausführung wäre in diesem Zusammenhang die Verwendung eines Rückschlagventil und/oder eines entfernbaren Ventils als Fluidkupplung vorstellbar.
Das Schliesselement eines Rückschlagventils ist vorzugsweise durch einen Mechanismus aus einem geschlossenen in einen offenen Zustand versetzbar.
Somit kann vorzugsweise der Durchfluss eines Fluids im offenen Zustand möglich sein und im geschlossenen Zustand durch den Druck des Fluids in zumindest eine Richtung geschlossen sein.

Die Fluidkupplung kann verbindbar an der Drahtvorrichtung anbringbar sein.
Insbesondere ist die Verwendung eines aufsteckbaren und/oder aufschraubbarem Ventils als Fluidkupplung vorstellbar. Bei der Anbringung einer solchen Fluidkupplung kann zudem eine Befestigungsvorrichtung vorgesehen sein, insbesondere Klemmen und/oder Schellen.

Das Fluid kann eine Flüssigkeit sein, beispielsweise Wasser, Salzwasser oder auch ein Gas. Somit ermöglicht das Einbringen eines Fluids in den Druckschlauch das Versteifen des Versteifungsabschnitts. Bevorzugt ist jedoch die Verwendung einer Flüssigkeit, da dies beim Entweichen weniger gefährlich für Menschen ist, als ein Gas, insbesondere Luft.

Die Drahtvorrichtung kann vorzugsweise zumindest teilweise ein im Röntgen sichtbares Material, insbesondere Metall, umfassen.

Bei minimal-invasiven Operationen mit Drahtvorrichtungen kann die Position der Drahtvorrichtung, insbesondere das eingeführte proximale Ende der Drahtvorrichtung, mit dem blossen Auge nicht einsehbar sein. Demnach muss vorzugsweise ein bildgebendes Verfahren verwendet werden, um die eingeführte Drahtvorrichtung sichtbar zu machen. Vorzugsweise wird ein Röntgenverfahren, wie die Fluoroskopie angewendet, damit vorzugsweise Echtzeitbildgebung der eingeführten Drahtvorrichtung gewährleistet werden kann.

Da die Abschwächung von Röntgenstrahlen durch den Massenschwächungskoeffizient gegeben ist, sollte ein Material mit Elementen einer hohen Ordnungszahl gewählt werden.

Da Kunststoff oftmals hauptsächlich aus Kohlenstoffverbindungen besteht, die Röntgenstrahlung schlecht absorbieren, sollte die Drahtvorrichtung stattdessen sichtbares Material wie modifizierten Kunststoff oder Metalle enthalten. Bereits kleine Mengen von beispielsweise Eisen können die Sichtbarkeit der Drahtvorrichtung gewährleisten.

Das proximale Ende der Drahtvorrichtung kann eine Spitze aufweisen. Die Spitze kann das proximale Ende der Drahtvorrichtung druckdicht abschliessen. Die Länge der Spitze liegt vorzugsweise in einem Bereich von 0,2 mm bis 15 cm.

Das proximale Ende der Drahtvorrichtung mit der Spitze ist gemäss der Erfindung der Teil, der als erstes in das Gefäss des Patienten eingeführt wird.

Die Länge der Spitze bezeichnet in diesem Zusammenhang die Länge der Spitze in Richtung der Längsachse der Drahtvorrichtung.

Die Spitze schliesst vorzugsweise den Druckschlauch der Drahtvorrichtung druckdicht ab.

Die Spitze kann mit dem Aussenschlauch bündig angeordnet werden und ebenfalls druckdicht mit dem Aussenschlauch verbunden sein.

Die Spitze ist vorzugsweise direkt angrenzend zum Versteifungsabschnitt angeordnet.
Dabei weist die Spitze in einer Ausführungsform im Wesentlichen dieselbe Querschnittsfläche auf wie der Versteifungsabschnitt. Es sind aber in Abhängigkeit des Verwendungszwecks auch grössere oder kleinere Querschnittsflächen als die des Versteifungsabschnitts vorstellbar.

Die Spitze oder ein Bereich der Spitze kann eine andere Elastizität, als die restlichen Bereiche der Drahtvorrichtung und/oder des Versteifungsabschnitts aufweisen.

Insbesondere kann die andere Elastizität der Spitze durch andere Armierung, Anordnung der Versteifungselemente, und/oder Ausbildung der torsionssteifen Struktur ausgebildet sein.
Die Spitze kann zudem eine variierende Elastizität in verschiedenen Bereichen der Spitze aufweisen.

Die Elastizität der Spitze kann in Abhängigkeit des Verwendungszwecks höher oder niedriger als die Elastizität des Versteifungsabschnitts ausgebildet sein.

Die Länge der Spitze ist bevorzugt möglichst kurz, also kleiner als 10% der Länge des Versteifungsaschnittes, ausgebildet. Somit ist vorzugsweise ein möglichst kleiner Bereich am proximalen Ende der Drahtvorrichtung nicht Teil des Versteifungsabschnitts.

In einer vorstellbaren Ausführungsform ist die Spitze ebenfalls zumindest teilweise versteifbar.

Die Spitze der Drahtvorrichtung kann zumindest teilweise konisch, abgerundet, sphärisch, elliptisch, kreiszylindrisch und/oder gestuft zylindrisch ausgebildet sein.

Diese Ausbildung der Spitze bietet den Vorteil, dass die Verletzungsgefahr bei Verwendung der Drahtvorrichtung minimiert wird. Beim Einführen der Drahtvorrichtung in einen Patienten verursacht eine solche Spitze der Drahtvorrichtung geringere oder keine Verletzungen und Schmerzen.

Zudem minimiert eine solche Spitze eine Verletzung von Gefässen und das potentielle Lösen von Ablagerungen oder Gerinnseln.

Aussen auf dem Aussenschlauch der Drahtvorrichtung kann eine hydrophile oder hydrophobe Beschichtung angeordnet sein.

Die Beschichtung des Aussenschlauchs kann unter anderem das Gleitverhalten verbessern und die Reibung minimieren. Auf der anderen Seite muss Biokompatibilität gewährleistet sein. Die Infektionsgefahr sollte gering gehalten werden, sowie Hämokompatibilität, Antithrombogenität und/oder geringe Partikelfreisetzung optimiert sein. Dazu eignen sich besonders gut Beschichtungen durch Polymere.

In den Beschichtungsverfahren wird die Oberfläche vorzugsweise vorher aktiviert, um Beschichtungen aufnehmen zu können. Dies kann beispielsweise durch die Plasmaaktivierung erfolgen, bei der ein Elektronenplasma dazu führt, dass offene Bindungsstellen auf der Oberfläche entstehen, die mit polaren Hydroxy-Gruppen besetzt werden. Somit entsteht eine hydrophile Oberfläche und die Polymerbeschichtung kann besser haften.

Die zu beschichtende Oberfläche kann beispielsweise durch Plasmapolymerisation im Vakuum durch Zuführen von Gas mit Precursor-Monomeren, die sich auf der Oberfläche ablagern, je nach Precursor-Monomeren, hydrophil oder hydrophob gemacht werden. Andere Beschichtungsverfahren wie Tauchbeschichtung, Spritzbeschichtung, Reel-to-Reel-Beschichtung sind ebenfalls vorstellbar.

In einem letzten Schritt können die Moleküle der Beschichtung noch stärker vernetzt werden, indem thermische Energie oder UV-Strahlung zugeführt wird.

Als hydrophobe Beschichtung für den Aussenschlauch eignet sich Polytetrafluorethylen, da das Molekül durch den symmetrischen Aufbau unpolar ist, eine sehr geringe Oberflächenspannung aufweist und geringe Kohäsions- und Adhäsionskräfte zu anderen Substanzen aufweist. Weiterhin wären auch anderes Polyfluorethylen, Hexafluorpropen und/oder Polydimethylsiloxan als hydrophobe Beschichtungen vorstellbar, wobei Polydimethylsiloxan sogar vorteilhafter Weise antithrombogen wirkt.

Als hydrophile Polymere eignen sich hingegen Beschichtungen, die Wasserstoffbrückenbindungen ausbilden können, sodass ein Wasserfilm leicht an die Oberfläche gebunden werden kann. Hydrophile Beschichtungen können nur aus einem hydrophilen Polymer bestehen, oder eine Kombination von hydrophilen Polymeren umfassen. Vorteilhafte vorstellbare hydrophile Beschichtungen sind unter anderem Hydrogele oder auch Polyvinylpyrrolidon, welches in seinen Eigenschaften durch Zugabe von Vinylpyrrolidon-Copolymere weiter angepasst werden kann.

Der Versteifungsabschnitt der Drahtvorrichtung kann einen Teil oder die gesamte Drahtvorrichtung bilden.

Die Länge und der Bereich des Versteifungsabschnitt kann vorteilhafterweise leicht auf die jeweiligen Problemstellungen angepasst sein. Besonders vorteilhaft ist eine Länge der Versteifungsschicht in einem Bereich von 2 cm bis 100 cm, insbesondere bevorzugt rund 30 cm. Der Versteifungsabschnitt ist vorzugsweise bis an das proximale Ende und/oder die Spitze ausgebildet.

Somit kann der Versteifungsabschnitt nur den Bereich einnehmen, der benötigt wird, um die Drahtvorrichtung in einem Bereich zu fixieren. Der Rest der Drahtvorrichtung kann von den Vorteilen einer fehlenden Versteifungsschicht profitieren, wie beispielsweise erhöhter Elastizität und/oder geringerer Schichtdicke. Somit können zudem Kosten reduziert werden und dennoch eine vollständige Funktionsweise gewährleistet werden.

Die gesamte Länge der Drahtvorrichtung ist vorzugsweise je nach gewünschtem Verwendungszweck zwischen 100 cm und 400 cm, insbesondere 160 cm und 260 cm, lang.

Der Aussenschlauch der Drahtvorrichtung kann einen Aussendurchmesser von maximal 0,035 Zoll, insbesondere 0,018 Zoll, insbesondere vorzugsweise 0,014 Zoll oder 0,012 Zoll, aufweisen.

Ein kleiner Aussendurchmesser ist für minimal-invasive Eingriffe oftmals vorteilhaft und ermöglicht auch neurologische Eingriffe.

Ein kleiner Aussendurchmesser ist notwendig, um in engen Gefässen Platz zu finden, und/oder Windungen von bis zu rund 360° mit der Drahtvorrichtung in Gefässen vollziehen zu können. Deshalb ist es ein besonders vorteilhaftes Merkmal der Erfindung, dass die Drahtvorrichtung selbst bei geringem Aussendurchmesser voll funktionsfähig ist und mit geringen Kosten hergestellt werden kann.

Der Druckschlauch der Drahtvorrichtung und die Versteifungsschicht können miteinander verbunden sein.

Der Druckschlauch und die Versteifungsschicht können gemeinsam radial bewegbar ausgebildet sein.
Vorzugsweise ist derartig ein Druck im Druckschlauch aufbaubar, dass der Druckschlauch und die Versteifungsschicht gemeinsam von innen gegen den Aussenschlauch drückbar sind.

Die Drahtvorrichtung kann als ein Führungsdraht und/oder ein Formdraht ausgebildet sein.

Die Drahtvorrichtung als Führungsdraht erlaubt es eine Krümmung für aufschiebbare Vorrichtungen vorzugeben.
Dazu wird die Drahtvorrichtung vorzugsweise im flexiblen Zustand verkrümmt und durch Druck mit einströmenden Fluid versteift.
Die aufschiebbare Vorrichtung kann entlang der durch den versteiften Führungsdraht vorgegebenen Krümmung aufgeschoben werden.

Die Drahtvorrichtung als Formdraht hingegen kann dazu ausgebildet sein, dass das proximale Ende der Drahtvorrichtung einer aufschiebbare Vorrichtung in den Gefässen eine Biegung vorgeben kann.

Es wäre vorstellbar, dass die Drahtvorrichtung als Formdraht einer aufschiebbaren Vorrichtung, insbesondere vorzugsweise einem Katheter und/oder einem Dilatator, eine Form vorgibt, bevorzugt im Bereich des proximalen Endes der aufschiebbaren Vorrichtung.

Es ist eine Ausführung vorstellbar, wobei die Drahtvorrichtung eine Biegung innerhalb der aufschiebbaren Vorrichtungen ausbilden kann und/oder ausbildet.

Dazu ist der Formdraht vorzugsweise in Abhängigkeit des Verwendungszwecks gebogen und/oder biegbar, und in die Drahtvorrichtung einführbar.

Dies hat den Vorteil, dass die aufschiebbare Vorrichtung weniger steif ausgebildet sein kann um die Drahtvorrichtung aufzunehmen. Die aufschiebbare Vorrichtung kann erst beim Versteifen der Drahtvorrichtung ebenfalls die vorbestimmte Form in einem Bereich einnehmen und/oder entlang dieser Form aufschiebbar sein.

Die Drahtvorrichtung als Formdraht und/oder Führungsdraht muss somit vorzugsweise nicht aus der aufschiebbaren Vorrichtung entfernt werden und/oder ausgetauscht werden.

Die Drahtvorrichtung kann vorzugsweise elastischer im unversteiften Zustand als die aufschiebbare Vorrichtung, oder ein Bereich der aufschiebbaren Vorrichtung, ausgebildet sein.
Die Drahtvorrichtung kann im versteiften Zustand weniger elastisch ausgebildet sein als die aufschiebbare Vorrichtung, oder ein Bereich der aufschiebbaren Vorrichtung.

Somit kann die Drahtvorrichtung vorzugsweise aus einer aufschiebbaren Vorrichtung im Wesentlichen ohne Verformung entfernt und/oder eingeführt werden.

Insbesondere weist die Drahtvorrichtung als Formdraht und/oder Führungsdraht Biegungen für die Führung auf und/oder ist zu Biegungen verformbar ausgebildet.
Vorzugsweise kann eine Biegung des Formdrahtes angepasst werden, sodass der Formdraht flexibel verwendet werden kann.

Die Drahtvorrichtung kann versteift werden, sobald die Drahtvorrichtung an einem Zielgefäss positioniert ist.

Die Drahtvorrichtung kann zudem versteift werden, wenn es zum Vollziehen eines bestimmten Pfads innerhalb der Gefässe zweckdienlich ist.
So wird ein sicherer Zugang für die eigentliche Behandlung des Zielgefässes ermöglicht.

Es wäre in diesem Zusammenhang vorstellbar, dass die Drahtvorrichtung als Formdraht in einer vorbestimmten Form versteifbar ausgebildet ist.

Mit einer Nadel kann ein Zugang in ein Zugangsgefäss geschaffen werden, sodass eine Drahtvorrichtung in ein Zielgefäss einführbar ist. Die Nadel kann zudem nach dem Einführen der Drahtvorrichtung wieder entfernt werden.

Die Aufgabe wird weiter durch ein Verfahren zum selektiven Versteifen einer Drahtvorrichtung, wie vorhergehend beschrieben, gelöst.
Durch Einbringen eines Fluids in den Druckschlauch kann die Drahtvorrichtung durch die Versteifungsschicht und den Aussenschlauch versteift werden.

Das selektive Versteifen der Drahtvorrichtung ermöglicht zum einen die benötigte Flexibilität zum Einführen in die Gefässe und zum anderen die selektive Stabilität einer versteiften Drahtvorrichtung.

Auch die Verwendungszeit wird minimiert, da eine Drahtvorrichtungen nach dem Stand der Technik oftmals von ihrem Verwendungsort, beispielsweise durch Blutfluss, das Manipulieren in der Drahtvorrichtung, oder Abrutschen der Drahtvorrichtung, ausgelenkt werden kann.

Ausserdem ist ein Fluid zum einen aus Sicherheitsgründen möglichst so zu wählen, dass selbst für den Fall einer starken Beschädigung der Drahtvorrichtung keine gesundheitlichen Schäden entstehen können. In diesem Zusammenhang wäre auch eine Vorrichtung zum Überwachen des Drucks durch das eingebrachte Fluid vorstellbar.

Durch Reduktion des Druckes im Innenraum im Druckschlauch kann die Drahtvorrichtung vorzugsweise wieder beweglich werden, indem die Versteifungsschicht nicht mehr gegen den Aussenschlauch gedrückt wird.

Vorzugsweise ist die Drahtvorrichtung bei einer Reduktion auf im Wesentlichen atmosphärischen Druck und Druck in den Blutgefässen eines Menschen im elastischen Zustand.

Besonders wichtig ist dabei, dass diese Versteifung selektiv aktiviert und deaktiviert werden kann.
Somit kann die Drahtvorrichtung flexibel eingeführt werden.
Am Zielort kann die Drahtvorrichtung für den minimal-invasiven operativen Eingriff, gemäss individueller Anatomie, versteift werden und anschliessend zum Entfernen wieder elastisch gemacht werden.
Somit können vorzugsweise mögliche gesundheitliche Schäden vermieden werden.
Mögliche Schäden, wie beispielsweise Schlaganfälle, können durch das Lösen von Ablagerungen oder Thromben, sowie eingeführtes Werkzeug und/oder Geräte und/oder Kleinstteile verursacht werden.
Durch eine zumindest teilweise versteifte Drahtvorrichtung werden diese Risiken minimiert und präziseres Arbeiten ermöglicht.

Es ist daher von besonderer Wichtigkeit diese potentiellen Schäden zu vermeiden und ein grosser Vorteil der Erfindung, dass selektives Versteifen der Drahtvorrichtung nach Bedarf durch das Einbringen eines Fluids in den Zwischenraum im Druckschlauch versteift werden kann.

Es ist zudem von Vorteil, dass der Druck nur aufgebaut werden muss um die Drahtvorrichtung zu versteifen und abgebaut werden muss um sie elastisch zu machen, da somit sichergestellt ist, dass die Drahtvorrichtung auch bei Versagen der Druckvorrichtung wieder lösbar ist.

Müsste ein Druck aufgebaut werden, um sie elastisch zu machen, wäre im Falle einer undichten Drahtvorrichtung eine versteifte Drahtvorrichtung in den Gefässen eines Menschen. Diese wäre nicht, oder nur sehr schwer, und/oder unter grossen Gefahren für den Menschen entfernbar.

Im Folgenden werden Ausführungsformen der Erfindung detailliert mit Bezugszeichen beschrieben. Hierbei zeigt:
- Figur 1:: einen Versteifungsabschnitt der Drahtvorrichtung,
- Figur 2:: ein Ausführungsbeispiel des Versteifungsabschnitts,
- Figur 3:: ein Ausführungsbeispiel des Versteifungsabschnitts,
- Figur 4:: ein Ausführungsbeispiel des Versteifungsabschnitts,
- Figur 5:: ein Ausführungsbeispiel des Versteifungsabschnitts,
- Figur 6:: ein Ausführungsbeispiel des Versteifungsabschnitts,
- Figur 7:: eine Struktur der Versteifungsschicht,
- Figur 8:: eine alternative Struktur der Versteifungsschicht,
- Figur 9:: einen Querschnitt des Versteifungsabschnitts der Drahtvorrichtung,
- Figur 10:: eine Drahtvorrichtung mit einer torsionssteifen Struktur im Profil,
- Figur 11:: eine Drahtvorrichtung mit einer torsionssteifen Struktur im Profil,
- Figur 12:: eine Drahtvorrichtung mit einem Versteifungsabschnitt mit partiell entfernten Schichten,
- Figur 13:: einen Längsschnitt eines Versteifungsabschnitts der Drahtvorrichtung mit einer Spitze,
- Figur 14:: einen Längsschnitt einer Drahtvorrichtung mit einem Versteifungsabschnitt und einer Spitze,
- Figur 15:: ein Ausführungsbeispiel einer Drahtvorrichtung als Formdraht und einer Drahtvorrichtung als Führungsdraht.

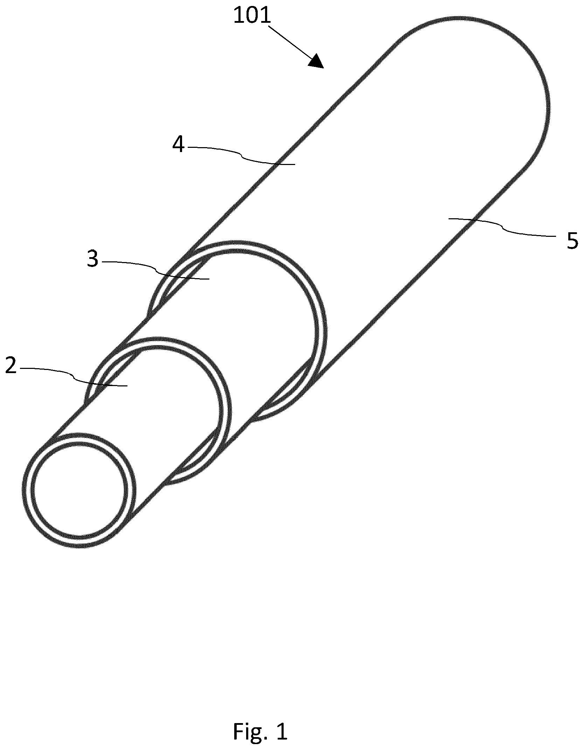

Figur 1 zeigt eine Ausführungsform eines Versteifungsabschnitts 101 der Drahtvorrichtung 100 mit drei Schlauchschichten. Die Drahtvorrichtung 100 ist zur besseren Sichtbarkeit in offenen Abschnitten der einzelnen Schichten dargestellt. Von aussen nach innen ist ein Aussenschlauch 4, eine Versteifungsschicht 3 und ein Druckschlauch 2 angeordnet.

Der Druckschlauch 2 wird zum Versteifen mit einer isotonischen Natriumchloridlösung mit einem Druck von 16 bar gefüllt. Somit drückt die Versteifungsschicht 3 gegen den Aussenschlauch 4, indem sie radial nach aussen bewegt wird. Beim Entfernen des Drucks wird die Versteifungsschicht 3 zudem wieder radial nach innen bewegt und die Versteifung des Versteifungsabschnitts 101 lässt nach.

Der Druckschlauch 2 ist in dieser Ausführungsform aus Thermoplasten gefertigt und kann somit durch das Einführen von einer isotonischen Natriumchloridlösung expandiert werden und elastisch radial verformt werden.

Die Versteifungsschicht 3 ist beim Entfernen des angelegten Drucks auch wieder radial nach innen bewegbar. Somit ist sichergestellt, dass die Drahtvorrichtung 100 immer entfernbar ist, insbesondere wenn der Druck nicht mehr aufgebaut werden kann.

Die Drahtvorrichtung 100 ist im Grundzustand ohne Druck beweglich ausgebildet, sodass keine Gefahr durch eine irreversible Versteifung der Drahtvorrichtung 100 bei einem Defekt besteht.

Die Versteifungsschicht 3 ist in diesem Fall aus einem losen, gegeneinander beweglichen, Geflecht aus Edelstahl und/oder Kunststoff gebildet, welches zu einem Teilvektor in Längsrichtung verläuft. Somit kann die Versteifungsschicht 3 leicht expandieren und es ist eine starke Reibung mit dem Aussenschlauch 4 herstellbar.

Der Aussenschlauch 4 umfasst in diesem Ausführungsbeispiel Polysiloxane und eine Edelstahlspirale, wobei die Edelstahlspirale helikal entlang der Längsachse der Drahtvorrichtung 100 ausgerichtet ist, im Polysiloxan eingebettet ist und vollständig umschlossen ist. In diesem Zusammenhang wäre aber auch die alternative Verwendung von Polyurethan für den Aussenschlauch 4 vorstellbar.

Der Aussenschlauch 4 ist zudem hydrophil durch eine Beschichtung 5 mit Polyvinylpyrrolidon. Somit ist das Einführen in einen Körpergang leichter möglich und atraumatisch durchführbar. Ausserdem werden durch die Beschichtung 5 die Gleiteigenschaften der Drahtvorrichtung 100 innerhalb der Gefässe erhöht.

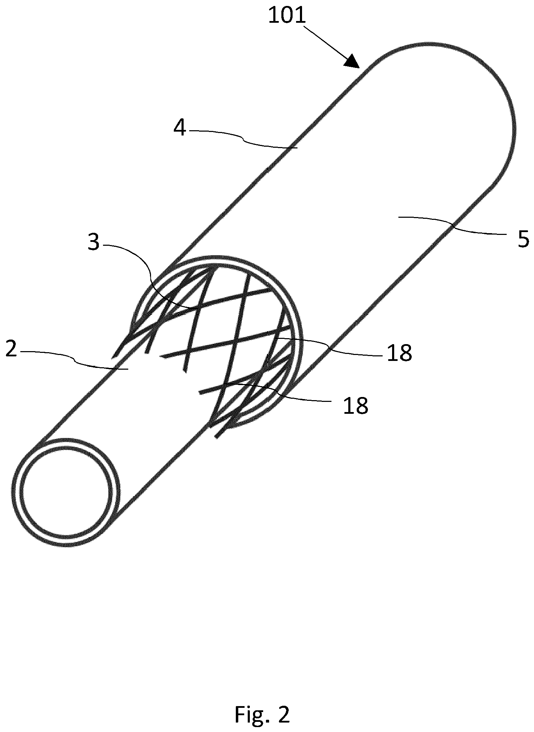

Figuren 2-5 zeigen explizite Ausführungsformen alternativ zu Fig. 1. Bei analogen Bezugszeichen, Funktionen und Ausbildungen der Erfindung, die nicht explizit erwähnt werden, wird auf Figur 1 verwiesen.

Figur 2 zeigt den Versteifungsabschnitt 101 der Drahtvorrichtung 100, die eine Versteifungsschicht 3 mit einem Geflecht aus Edelstahl zeigt, wobei die einzelnen Stabilisierungselemente 18 der Versteifungsschicht 3 gegeneinander verschiebbar gefertigt sind. Die Versteifungsschicht 3 ist durch den Druckschlauch 2 radial nach aussen bewegbar und kann durch Reibung mit dem Aussenschlauch 4 den Versteifungsabschnitt 101 versteifen. Es sind aber auch andere Materialien für die Stabilisierungselemente 18 wie zum Beispiel Polyamid vorstellbar.

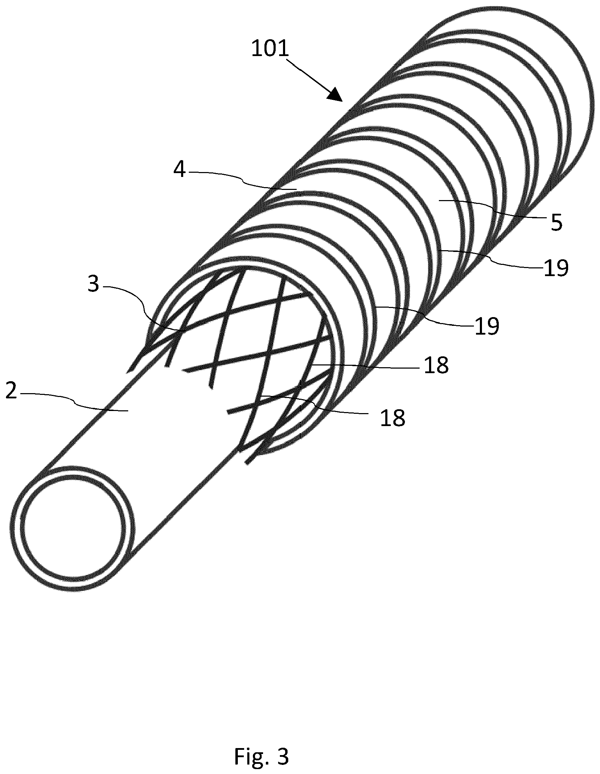

Figur 3 zeigt ein Ausführungsbeispiel des Versteifungsabschnitts 101 der Drahtvorrichtung 100 analog zu Figur 2.
Die Drahtvorrichtung 100 weist ein Geflecht aus Edelstahl der Versteifungsschicht 3 mit Stabilisierungselementen 18 auf. Zudem weist der Aussenschlauch 4 eine Armierung 19 aus Edelstahl auf. Die Armierung 19 ist dabei möglichst konzentrisch um die Längsachse der Drahtvorrichtung 100 ausgebildet, sodass die Drahtvorrichtung 100 weiterhin möglichst flexibel gegenüber Biegung senkrecht zur Längsachse der Drahtvorrichtung ausgebildet ist, wenn sie sich nicht im versteiften Zustand befindet. Die Armierung 19 ist in diesem Ausführungsbeispiel durch Ringe realisiert, aber auch eine spiralförmige Armierung 19 und eine maschenförmige Armierung 19 sind vorstellbar.

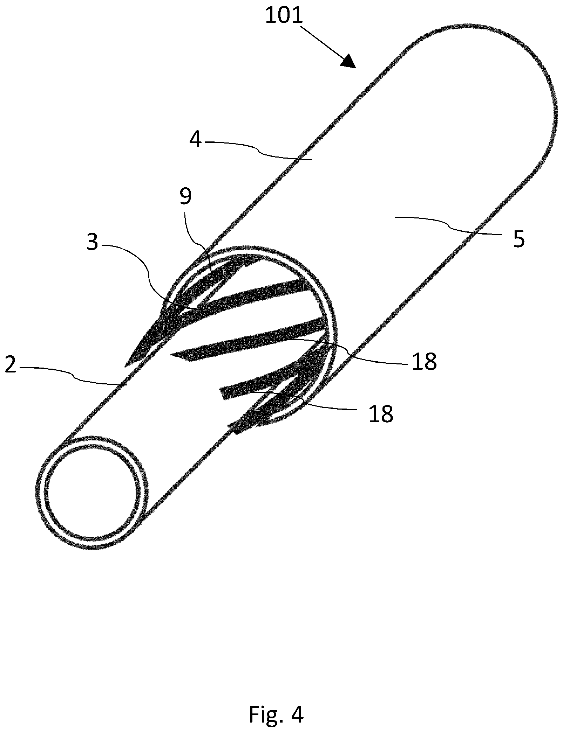

Figur 4 zeigt ein Ausführungsbeispiel des Versteifungsabschnitts 101 der Drahtvorrichtung 100.
Die Stabilisierungselemente 18 sind entlang genau einer helikalen Ausrichtung um den Druckschlauch 2 angeordnet.
Durch eine solche helikale Ausrichtung der Stabilisierungselemente 18 wird eine torsionssteife Struktur 9 ausgebildet.

Die torsionssteife Struktur 9 der Versteifungsschicht 3 kann somit in eine Drehrichtung um die Längsachse des Versteifungsabschnitts 101 Drehmoment entlang des gesamten Versteifungsabschnitts 101 und/oder der Drahtvorrichtung 100 (nicht vollständig gezeigt in Fig. 4) übertragen.

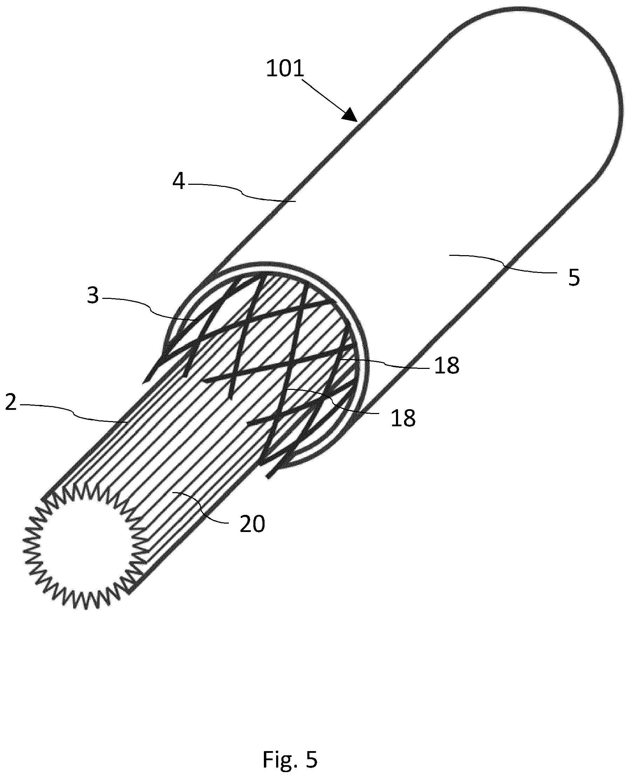

Figur 5 zeigt ein Ausführungsbeispiel des Versteifungsabschnitts 101 der Drahtvorrichtung 100, wobei der Druckschlauch 2 durch Entfaltung einer gefalteten Struktur 20, die Versteifungsschicht 3 und die Stabilisierungselemente 18 radial nach aussen gegen den Aussenschlauch 4 drücken kann. Der maximale Aussendurchmesser des Druckschlauches 2 nach dem Auffalten ist grösser als der Innendurchmesser des Aussenschlauches 4, so dass die Versteifungsschicht 3 gegen den Aussenschlauch 4 drückbar ist. Somit verändert sich die Ausdehnung des Druckschlauches 2 auch nach mehrmaliger Verwendung nicht, da keine oder im Wesentlichen keine elastische Verformung stattfindet. Die gefaltete Struktur 20 des Druckschlauchs 2 faltet sich auf, sobald mit dem Druckschlauch 2 ein Druck durch das Einführen der Salzlösung angelegt wird und faltet sich wieder ein, wenn der Druck entfernt wird. Dies hat zudem den Vorteil, dass die elastische Hysterese durch die gefaltete Struktur 20 minimiert wird. Es wird also minimiert, dass das Verbleiben einer Deformation nach dem Entfernen der auslenkenden Kraft fortbesteht. Somit wird die Sicherheit, sowie die Haltbarkeit des Druckschlauchs 2 der Drahtvorrichtung 100 gewährleistet.

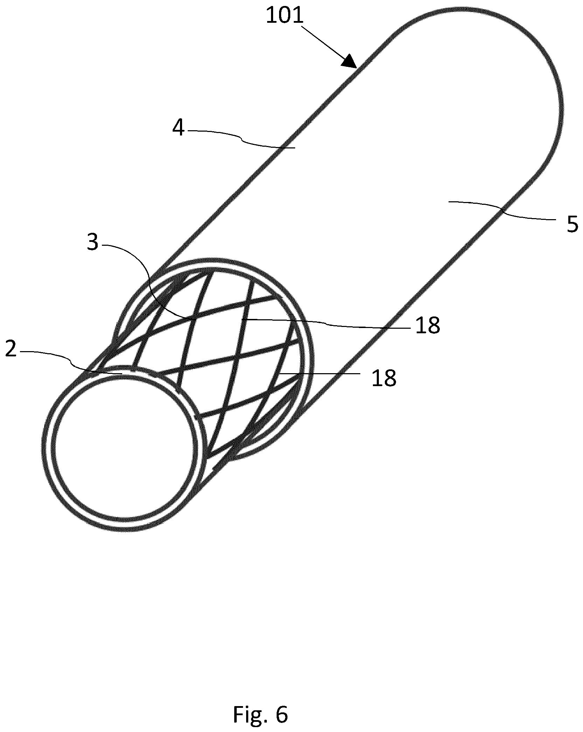

Figur 6 zeigt ein Ausführungsbeispiel des Versteifungsabschnitts 101 der Drahtvorrichtung 100, wobei der Druckschlauch 2 direkt an die Versteifungsschicht 3 angrenzt und mit der Versteifungsschicht 3 verbunden ist. Der Druckschlauch 2 und die Versteifungsschicht 3 bilden einen druckdichten Schlauch mit Stabilisierungselementen 18 aus.

Der Druckschlauch 2 ist somit zusammen mit der Versteifungsschicht 3 radial bewegbar und kann durch einen angelegten Druck gemeinsam mit der Versteifungsschicht 3 radial nach aussen gegen den Aussenschlauch 4 gedrückt werden, um den Versteifungsabschnitt 101 zu versteifen.

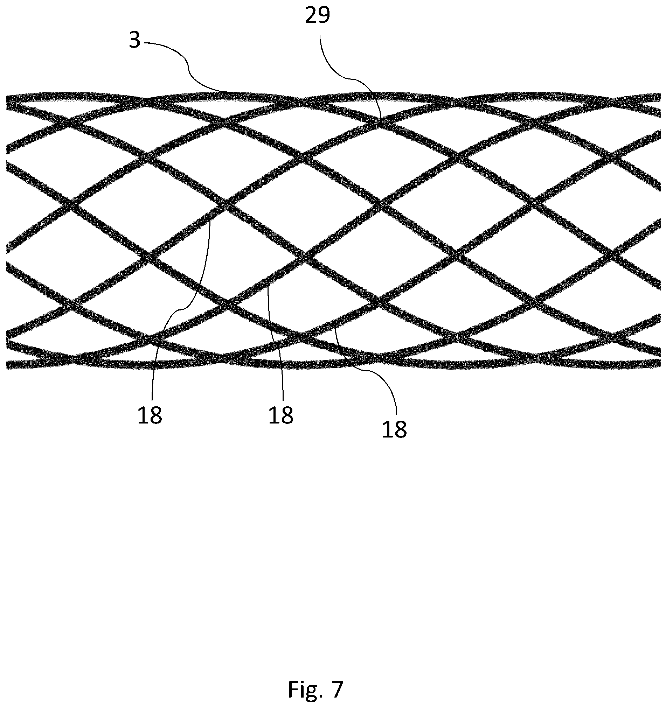

Figur 7 zeigt ein Ausführungsbeispiel der Versteifungsschicht 3 mit Stabilisierungselementen 18, wobei das gebildete Geflecht an den Schnittstellen 29 nicht starr miteinander verbunden ist, sondern gegeneinander frei beweglich ausgebildet ist, um radiale Verformbarkeit zu gewährleisten. Die Stabilisierungselemente 18 sind in diesem Ausführungsbeispiel in einem Winkel von im Wesentlichen 30° zur Längsachse der Drahtvorrichtung 100 ausgebildet.

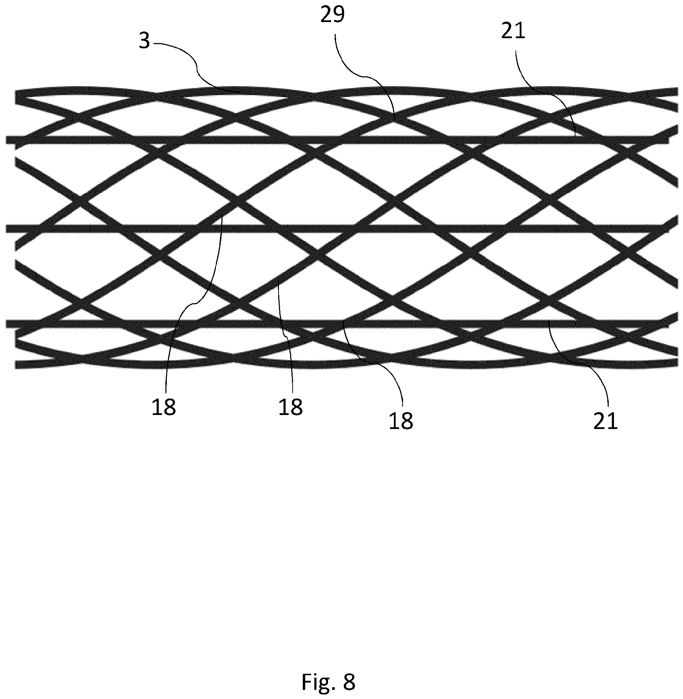

Figur 8 zeigt ein weiteres Ausführungsbeispiel der Versteifungsschicht 3 mit Stabilisierungselementen 18 analog zu Figur 7, wobei zusätzlich longitudinale Stabilisierungselemente 21 im Wesentlichen parallel zur Längsachse dargestellt sind, die die Reibung zusätzlich verstärken, wenn sie radial gegen den Aussenschlauch 4 gedrückt werden.

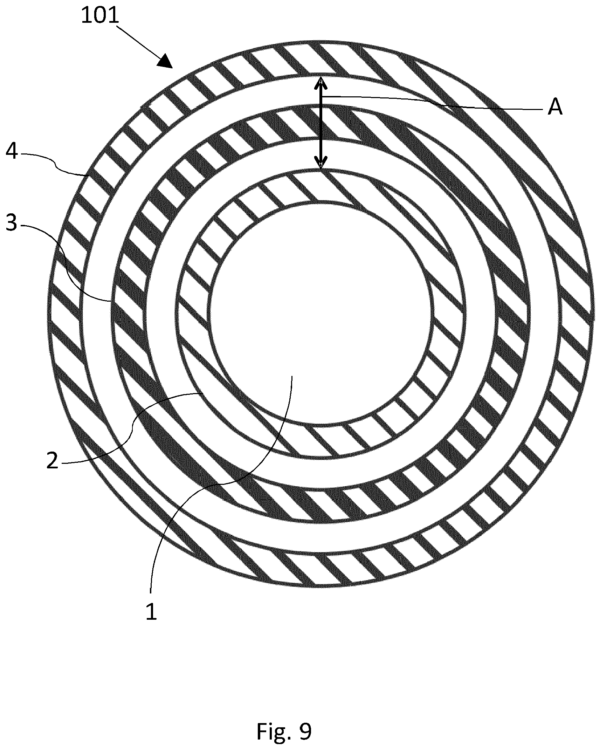

Figur 9 zeigt einen Querschnitt des Versteifungsabschnitts 101 der Drahtvorrichtung 100 mit einem Druckschlauch 2, einer Versteifungsschicht 3 und einem Aussenschlauch 4. Von aussen nach innen ist ein Aussenschlauch 4, eine Versteifungsschicht 3, ein Druckschlauch 2 und ein Innenraum 1 zu sehen. Zudem wurde der Bereich A zwischen dem Druckschlauch 2 und dem Aussenschlauch 4, zwischen denen der Druck eine radiale Bewegung verursachen kann, markiert. Durch den angelegten Druck im Druckschlauch 2 durch einströmendes Fluid ist eine radiale Bewegung der Versteifungsschicht 3 nach aussen ausführbar, die die Versteifungsschicht 3 auf den Aussenschlauch 4 drückt und somit versteift. Die Wahl der Materialien und die Funktionsweise ist ansonsten analog zu Figur 1.

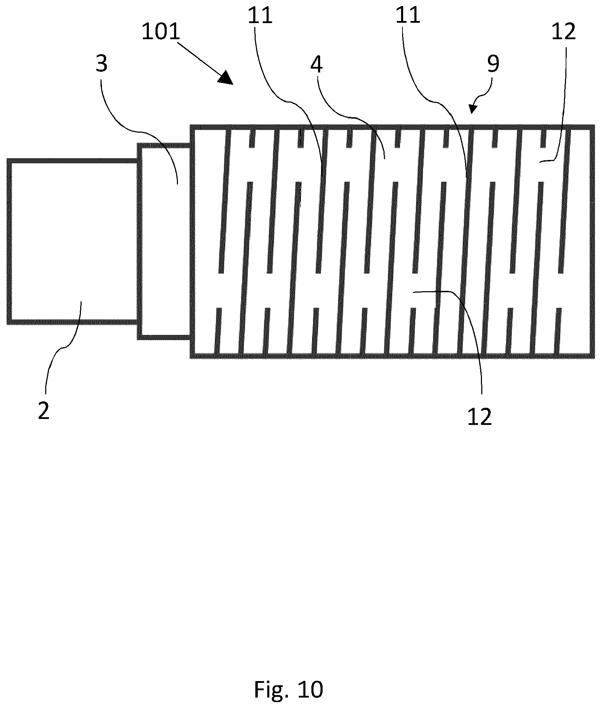

Figur 10 zeigt eine Drahtvorrichtung mit torsionssteifer Struktur 9 die im Aussenschlauch 4 ausgebildet ist.

Die Drahtvorrichtung 100 ist zur besseren Sichtbarkeit in offenen Abschnitten der einzelnen Schichten im Profil dargestellt. Von aussen nach innen ist der Aussenschlauch 4, die Versteifungsschicht 3 und der Druckschlauch 2 abgebildet.
Der Aussenschlauch 4 besteht aus einem Hypotube vorwiegend aus Metall, welcher helikale Schnitte 11 entlang der Längsachse der Drahtvorrichtung 100 aufweist.
Der Aussenschlauch 4 ist radial im Wesentlichen unbeweglich ausgebildet.
Die helikalen Schnitte 11 entlang des Aussenschlauchs 4 bilden die torsionssteife Struktur 9 aus. Die helikalen Schnitte 11 sind in dieser Ausführung entlang einer Drehrichtung um die Längsachse angeordnet. Die helikalen Schnitte 11 verlaufen nicht durchgängig entlang der Oberfläche des Aussenschlauchs 4, sondern weisen Materialbrücken 12 auf. Die helikalen Schnitte 11 erstrecken sich in dieser Ausführung jeweils um nahezu eine Umdrehung um den Aussenschlauch 4 und sind alternierend mit versetzten helikalen Schnitten 11 angeordnet.

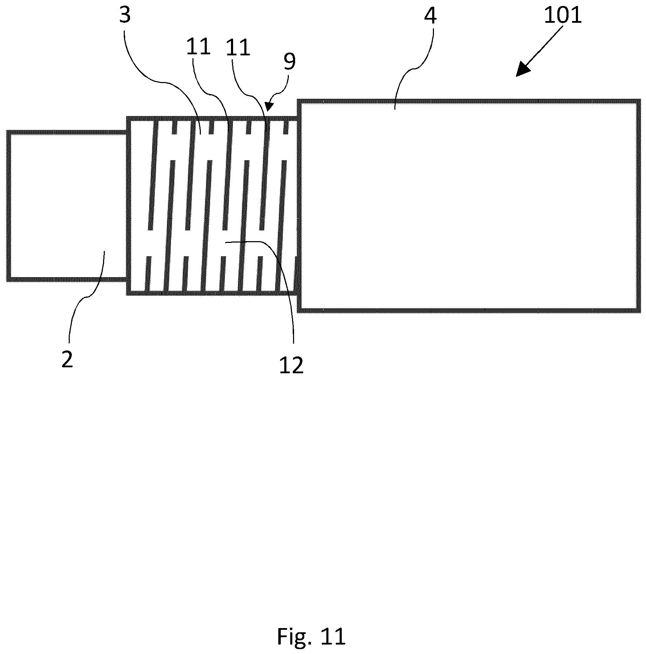

Figur 11 zeigt eine alternative Ausführung der Drahtvorrichtung 100 mit torsionssteifer Struktur 9 die in der Versteifungsschicht 3 ausgebildet ist. Die torsionssteife Struktur 9 auf der Versteifungsschicht 3 ist radial beweglich ausgebildet.
Die Wahl der Materialien und Funktionsweise ist ansonsten analog zu Figur 10 und es wird auf erneute Beschreibung verzichtet.

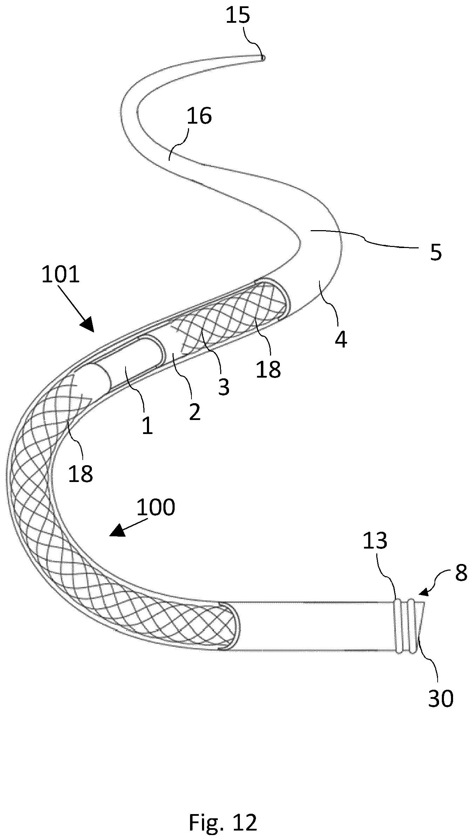

Figur 12 zeigt eine Drahtvorrichtung 100 mit einer Spitze 16 und einer Fluidkupplung 8. Der Aussenschlauch 4, die Versteifungsschicht 3 und der Druckschlauch 2 des Versteifungsabschnitts 101 wurden zur besseren Anschauung partiell entfernt.
Die Spitze 16 am proximalen Ende 15 in Fig. 12 schliesst direkt mit dem Aussenschlauch 4 ab und ist zur Anschaulichkeit verjüngt dargestellt. Dabei handelt es sich nur um eine perspektivische Darstellungsform. Der Aussenschlauch 4 ist durch eine Beschichtung 5 mit Polyvinylpyrrolidon hydrophil ausgebildet. Die Fluidkupplung 8 weist ein Gewinde 13 zur Anbringung einer Druckvorrichtung (nicht in Fig. 12 gezeigt) auf.
Die Fluidkupplung 8 ermöglicht in Kombination mit der Druckvorrichtung das Einführen von Fluid zum Versteifen des Versteifungsabschnitts 101. Die Fluidkupplung 8 am distalen Ende 30 weist ein Rückschlagventil auf (nicht in Fig. 12 gezeigt), sodass die Druckvorrichtung nach dem Anlegen von Druck entfernt werden kann, ohne dass es zu einem Druckverlust und/oder Entweichen von Fluid kommt. Der Druckschlauch 2 umgibt einen Innenraum 1, in welchen das Fluid einführbar ist. Zum Ablassen von Druck und Fluid weist die Fluidkupplung 8 eine Lösevorrichtung am Rückschlagventil auf (nicht in Fig. 12 gezeigt) auf. Die Lösevorrichtung ist durch eine Seele, die in die Fluidkupplung eingeführt werden kann in Sperrrichtung freigebbar ausgebildet, sodass ein Durchfluss in beide Richtungen möglich ist. Auf diese Weise ist der Druck entfernbar und der Versteifungsabschnitt 101 kann wieder flexibel beweglich gemacht werden.

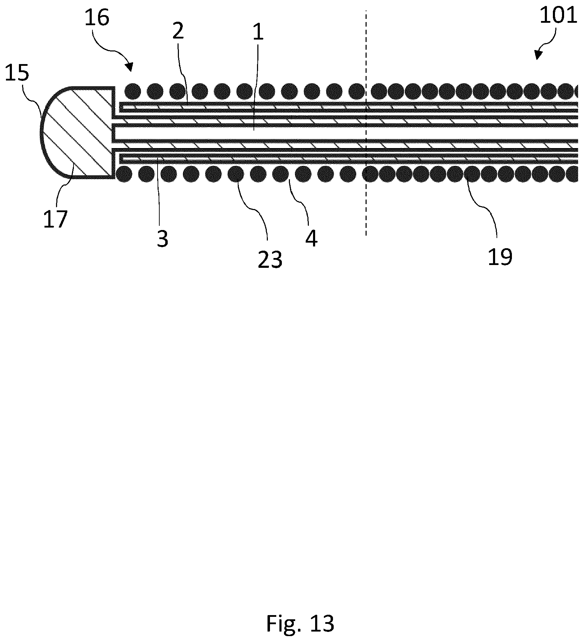

Figur 13 zeigt einen Längsschnitt der Spitze 16 einer Drahtvorrichtung 100. Die Spitze 16 ist zum proximalen Ende 15 druckdicht mit dem Druckschlauch 2 benachbart und weist eine abgerundete Form 17 auf. Die abgerundete Form 17 ist in dieser Ausführung aus elastischem Polysiloxan ausgebildet. Die Spitze weist im Wesentlichen denselben Querschnitt, wie die Versteifungsschicht 101 und der Aussenschlauch 4 auf. Die Versteifungsschicht 3 und der Innenraum 1 sind ebenfalls entlang eines versteifbaren Bereichs der Spitze 16 ausgebildet. Die Spitze 16 weist einen Bereich mit einer spiralförmigen Armierung 23 auf und bildet eine Fortsetzung des Versteifungsabschnitts 101 mit einer anderen spiralförmigen Armierung 19 aus. Die gestrichelte schwarze Linie veranschaulicht den Übergang vom Versteifungsabschnitt 101 mit anderer Armierung 19 zur Spitze 16 mit der schwächeren Armierung 23. Im Bereich der Spitze 16 ist dem Material weiterhin Eisenstaub zugesetzt, sodass Bewegungen unter Röntgenverfahren sichtbar gemacht werden kann.

Der versteifbare Abschnitt der Spitze 16 weist eine hohe Elastizität auf, da die Wicklung der Armierung 23 eine grössere Beabstandung aufweist als die Wicklung der Armierung 19. Somit weist der Versteifungsabschnitt 101 zum proximalen Ende 15 eine erhöhte Elastizität auf, was die Biegung der Drahtvorrichtung 100 in den Gefässen erleichtert. Der Aussenschlauch 4 wurde in Fig. 13 zur besseren Anschaulichkeit nicht als durchgängige Fläche dargestellt, ist aber druckdicht ausgebildet. Die Armierungen 19, 23 bestehen aus Edelstahl.

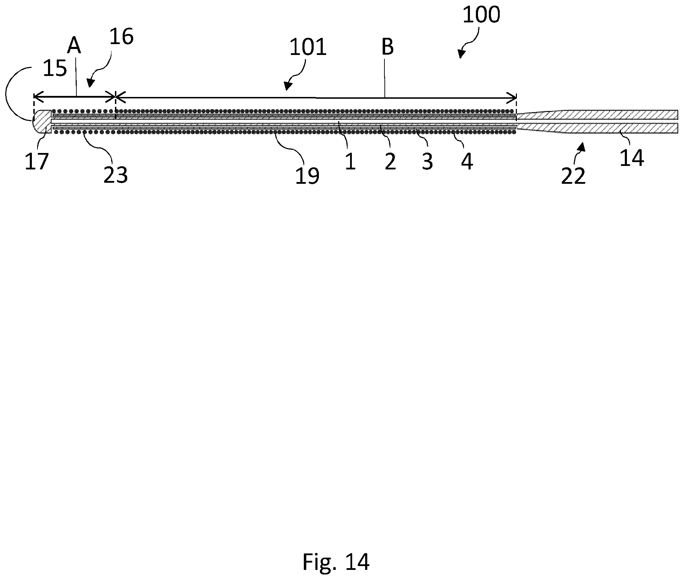

Figur 14 zeigt den Längsschnitt einer Drahtvorrichtung 100 mit einer Spitze 16 und Versteifungsabschnitt 101.
Auf wiederholte Beschreibungen wird verzichtet und auf Fig. 13 verwiesen. Der Bereich der Spitze 16 mit einer anderen spiralförmigen Armierung 23 und der abgerundeten Form 17 wurde mit A markiert. In diesem Zusammenhang wäre ebenfalls eine variierende Elastizität in verschiedenen Bereichen der Spitze 16 vorstellbar. Der Bereich des Versteifungsabschnitts 101 mit anderer Armierung 19 wurde mit B markiert.

Der Versteifungsabschnitt 101 ist benachbart zu einer unversteifbaren Schlauchvorrichtung 22 angeordnet. Diese unversteifbare Schlauchvorrichtung 22 weist eine Fortführung des Innenraums 1 und lediglich eine koaxiale Schlauchschicht 14 auf. Die koaxiale Schlauchschicht 14 weist eine höhere Schichtdicke auf und ist zum Versteifungsabschnitt 101 konisch ausgebildet und druckdicht mit dem Druckschlauch 2 ausgebildet. Die in Fig. 14 dargestellte Drahtvorrichtung 100 ist entlang ihrer gesamten Länge Drehmoment erhaltend ausgebildet da sie eine torsionssteife Struktur 9 (nicht in Fig. 14 gezeigt) umfasst, die sich entlang der gesamten Längsachse der Drahtvorrichtung 100 ausgebildet ist.

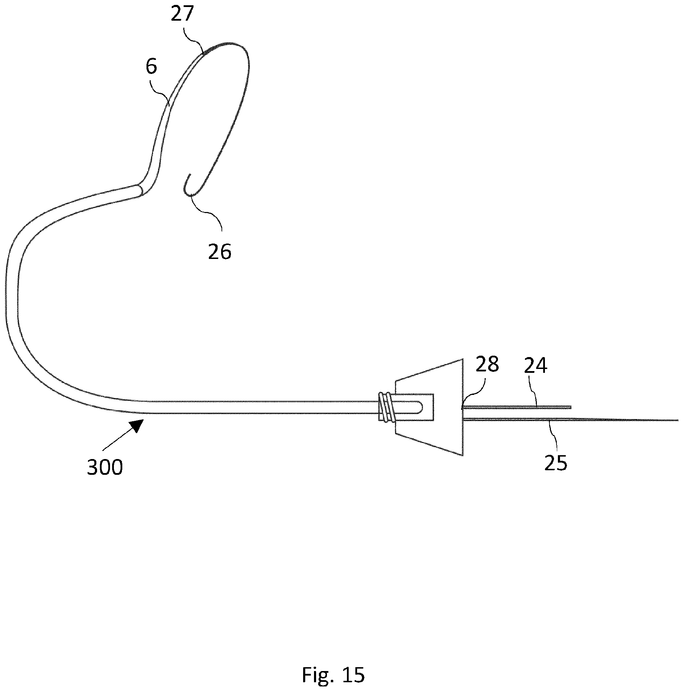

Figur 15 zeigt eine Ausführung einer aufschiebbaren Vorrichtung 300 die mit zwei Drahtvorrichtungen 100 formbar und führbar ausgebildet ist. Die aufschiebbare Vorrichtung 300 weist zwei separate Lumen auf mit jeweils einer Öffnung einem distalen Ende 28 der aufschiebbaren Vorrichtung 300 auf. In diese Öffnungen ist jeweils eine Drahtvorrichtung 100 einführbar. In dieser Ausführung weist ein Lumen zum proximalen Ende 27 der aufschiebbaren Vorrichtung 300 eine Öffnung auf, sodass die Drahtvorrichtung 100 als Führungsdraht 24 verwendet werden kann. Die Drahtvorrichtung 100 als Führungsdraht 24 kann eine Krümmung 26 ausbilden und ist in dieser gekrümmten Ausrichtung versteifbar durch den Versteifungsabschnitt 101 (nicht gezeigt in Fig. 15) ausgebildet. Durch diese Krümmung 26 kann eine Biegung innerhalb der Gefässe vorgegeben werden. Die aufschiebbare Vorrichtung 300 kann entlang dieser vorgegebenen Krümmung 26 aufgeschoben werden und ist entlang der Krümmung relativ zum Führungsdraht 24 in Gefässen bewegbar.

Die Drahtvorrichtung 100 als Formdraht 25 ist in das andere Lumen einführbar. Das andere Lumen ist zum proximalen Ende 27 der aufschiebbaren Vorrichtung 300 geschlossen, sodass der aufschiebbaren Vorrichtung 300 im Bereich 6 des proximalen Endes 27 eine Form durch die Drahtvorrichtung 100 als Formdraht 25 vorgegeben werden kann.

Die Drahtvorrichtung 100 als Formdraht 25 gibt in dieser Ausführungsform nur eine Form vor, wenn im Bereich 6 des proximalen Endes 27 der Versteifungsabschnitt 101 versteift wird.

## Patentansprüche

1. Drahtvorrichtung (100), insbesondere Drahtvorrichtung zum Einführen in einen Körpergang, umfassend einen Versteifungsabschnitt (101), umfassend einen Druckschlauch (2), eine Versteifungsschicht (3) und einen Aussenschlauch (4), wobei die Versteifungsschicht (3) konzentrisch um den Druckschlauch (2) angeordnet ist, und der Aussenschlauch (4) konzentrisch ausserhalb der Versteifungsschicht (3) angeordnet ist, wobei der Aussenschlauch (4) radial steif ausgebildet ist und die Versteifungsschicht (3) radial bewegbar, ausgebildet ist, **dadurch gekennzeichnet, dass** im Druckschlauch (2) derartig ein Druck aufbaubar ist, dass die Versteifungsschicht (3) von innen gegen den Aussenschlauch (4) drückbar ist und so unter Druck der Versteifungsabschnitt (101) reversibel versteifbar ist und insbesondere der Druck im Druckschlauch abbaubar ist, so dass der Versteifungsabschnitt wieder flexibel ist.

2. Drahtvorrichtung (100) nach Anspruch 1, **dadurch gekennzeichnet, dass** der Druckschlauch (2) erweiterbar, insbesondere elastisch und/oder auffaltbar ausgebildet ist.

3. Drahtvorrichtung (100) nach einem der vorhergehenden Ansprüchen, **dadurch gekennzeichnet, dass** die Versteifungsschicht (3) zumindest zwei Stabilisierungselemente (18) umfasst, wobei die Ausrichtung der Stabilisierungselemente (18) zumindest einen Teilvektor in Längsrichtung der Drahtvorrichtung (100) aufweist und die Stabilisierungselemente (18) vorzugsweise gegeneinander verschiebbar sind.

4. Drahtvorrichtung (100) nach Anspruch 3, **dadurch gekennzeichnet, dass** die Stabilisierungselemente (18) aus Metall und/oder Kunststoff bestehen und insbesondere eine Vielzahl von Stabilisierungselementen (18) zu einem Geflecht verflochten ist.

5. Drahtvorrichtung (100) nach einem der vorhergehenden Ansprüche, **dadurch gekennzeichnet, dass** der Aussenschlauch (4) druckdicht ausgebildet ist und insbesondere aus einem Kunststoff und/oder Metall hergestellt ist.

6. Drahtvorrichtung (100) nach einem der vorhergehenden Ansprüche **dadurch gekennzeichnet, dass** die Drahtvorrichtung (100) eine torsionssteife Struktur (9) umfasst, bevorzugt einen Hypotube, eine helikale und/oder verdrillte Struktur.

7. Drahtvorrichtung (100) nach einem der vorhergehenden Ansprüche, **dadurch gekennzeichnet, dass** die Drahtvorrichtung (100) eine Fluidkupplung (8) umfasst, durch die ein Fluid in den Druckschlauch (2) einbringbar ist, wobei insbesondere ein Querschnitt durch die Fluidkupplung (8) maximal dieselbe Ausdehnung wie ein Querschnitt des Versteifungsabschnitts (101) aufweist.

8. Drahtvorrichtung (100) nach einem der vorhergehenden Ansprüche, **dadurch gekennzeichnet, dass** die Drahtvorrichtung (100) zumindest teilweise ein im Röntgen sichtbares Material, insbesondere Metall, umfasst.

9. Drahtvorrichtung (100) nach einem der vorhergehenden Ansprüche, **dadurch gekennzeichnet, dass** ein proximales Ende (15) der Drahtvorrichtung (100) eine Spitze (16) aufweist, welche das proximale Ende (15) der Drahtvorrichtung (100) druckdicht abschliesst, wobei die Länge der Spitze (16) vorzugsweise im Bereich von 0,2 mm bis 15 cm liegt.

10. Drahtvorrichtung (100) nach einem der vorhergehenden Ansprüche, **dadurch gekennzeichnet, dass** aussen auf dem Aussenschlauch (4) eine hydrophile oder hydrophobe Beschichtung (5) angeordnet ist.

11. Drahtvorrichtung (100) nach einem der vorhergehenden Ansprüche, **dadurch gekennzeichnet, dass** der Versteifungsabschnitt (101) einen Teil oder die gesamte Drahtvorrichtung (100) bildet.

12. Drahtvorrichtung (100) nach einem der vorhergehenden Ansprüche **dadurch gekennzeichnet, dass** der Aussenschlauch (4) einen Aussendurchmesser von maximal 0,035 Zoll, insbesondere 0,018 Zoll, insbesondere vorzugsweise 0,014 Zoll oder 0,012 Zoll, aufweist.

13. Drahtvorrichtung (100), nach einem der vorhergehenden Ansprüche, **dadurch gekennzeichnet, dass** der Druckschlauch (2) und die Versteifungsschicht (3) miteinander verbunden sind.

14. Verfahren zum selektiven Versteifen einer Drahtvorrichtung (100) nach einem der vorhergehenden Ansprüche, **dadurch gekennzeichnet, dass** durch Einbringen eines Fluids in den Druckschlauch (2) durch die Versteifungsschicht (3) und den Aussenschlauch (4) die Drahtvorrichtung (100) versteift wird.

15. Verfahren nach Anspruch 14, **dadurch gekennzeichnet, dass** durch Reduktion des Druckes im Druckschlauch (2) die Drahtvorrichtung (100) wieder beweglich wird.
